(19) Europäisches Patentamt
European Patent Office
Office européen des brevets

(11) **EP 1 610 848 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**04.11.2009 Patentblatt 2009/45**

(45) Hinweis auf die Patenterteilung:
**04.10.2006 Patentblatt 2006/40**

(21) Anmeldenummer: **04763548.7**

(22) Anmeldetag: **28.07.2004**

(51) Int Cl.:
**A61M 5/315** (2006.01)

(86) Internationale Anmeldenummer:
**PCT/EP2004/008422**

(87) Internationale Veröffentlichungsnummer:
**WO 2005/046770 (26.05.2005 Gazette 2005/21)**

(54) **INJEKTIONSGERÄT**

INJECTION DEVICE

APPAREIL D'INJECTION

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**HR LT LV**

(30) Priorität: **03.11.2003 DE 20317377 U**

(43) Veröffentlichungstag der Anmeldung:
**04.01.2006 Patentblatt 2006/01**

(73) Patentinhaber: **Haselmeier GmbH**
**9000 St. Gallen (CH)**

(72) Erfinder:
• **GABRIEL, Jochen**
**70192 Stuttgart (DE)**
• **KEITEL, Joachim**
**73728 Esslingen (DE)**

(74) Vertreter: **Raible, Tobias et al**
**Raible & Raible**
**Schoderstrasse 10**
**70192 Stuttgart (DE)**

(56) Entgegenhaltungen:
**EP-A- 0 897 728**    **EP-A2- 0 327 910**
**WO- -03//080160**    **WO-A-00/41754**
**WO-A-03/086512**    **WO-A1-01//95959**
**WO-A1-05//018721**    **WO-A2-03//011370**
**US- - 5 295 976**    **US- - 5 938 642**
**US-A- 5 320 609**    **US-A- 5 626 566**
**US-A- 6 004 297**

**EP 1 610 848 B2**

**Beschreibung**

Die Erfindung betrifft ein Injektionsgerät, in welchem eine Kolbenstange in axialer Richtung geführt ist.

Bei Injektionsgeräten wird gewünscht, dass ihre Bedienung leicht verständlich, also intuitiv, ist, und dass der Patient eine gute Kontrolle über den Injektionsvorgang hat, also verstehen kann, was vor sich geht. Das Dokument WO 01/95959 offenbart ein Injektionsgerät entsprechend der Präambel des Anspruchs 1.

Es ist deshalb eine Aufgabe der Erfindung, ein neues Injektionsgerät bereit zu stellen.

Nach der Erfindung wird diese Aufgabe gelöst durch ein Injektionsgerät nach Anspruch 1. Vor einer Injektion stellt der Patient eine gewünschte Injektionsdosis ein, indem er das Gewindeteil relativ zur Kolbenstange und relativ zum Gehäuse verdreht, wobei sich das Gewindeteil relativ zur Kolbenstange und relativ zum Gehäuse axial verschiebt. Anschließend führt der Patient, nach dem Einstechen der Nadel, die Injektion durch, wobei sich das Gewindeteil relativ zum Gehäuse axial in Injektionsrichtung verschiebt und sich dabei zusammen mit der Kolbenstange bewegt, also keine Relativbewegung relativ zu dieser durchführt, weil es an einer Drehung relativ zu ihr gehindert wird. Man verwendet also ein geschicktes Zusammenspiel von Drehbewegungen und Axialbewegungen, um einesteils die Dosis einzustellen, und um andererseits nach dem Einstellvorgang die zuvor eingestellte Dosis zu injizieren. Eine solche Bedienung ist intuitiv leicht verständlich.

Falls der Patient die Dosis versehentlich zu hoch eingestellt hat, kann er sie wieder reduzieren. Diese "Dosiskorrektur" ist bei einem Gerät nach der Erfindung genauso einfach wie die Einstellung der Dosis selbst, und sie ist leicht zu verstehen.

Weitere Einzelheiten und vorteilhafte Weiterbildungen der Erfindung werden im Folgenden anhand von Ausführungsbeispielen erörtert, die insofern nicht patentgemäß sind, als dort die erste Antriebsverbindung zwischen dem Schieber (66) und dem Gehäuse (50) nicht in erfindungsgemäßer Weise als zweites Gewinde, sondern als axiale Führung (64, 70) ausgebildet ist, und zugleich die zweite Antriebsverbindung zwischen Schieber (66) und Einstellglied (76) nicht als patentgemäße axiale Führung, sondern als zweites Gewinde (86, 94) ausgeführt ist. Die in Anspruch 1 geschützte Erfindung legt insofern gegenüber den dargestellten nicht patentgemäßen Ausführungsformen eine kinematische Umkehr zugrunde.

Fig. 1    eine Seitenansicht des Gehäuses eines Injektionsgeräts nach einer bevorzugten Ausführungsform der Erfindung,

Fig. 2    einen Axialschnitt durch das Gehäuse der Fig. 1, gesehen längs der Linie II-II der Fig. 3,

Fig. 3    eine Ansicht, gesehen in Richtung des Pfeiles III der Fig. 2,

Fig. 4    eine Seitenansicht eines zur Dosiseinstellung dienenden Einstellglieds, welches auch als Skalenrohr bezeichnet wird,

Fig. 5    einen Längsschnitt durch das Einstellglied der Fig. 4,

Fig. 6    eine Draufsicht auf einen Schieber, der bei dieser Ausführungsform mit einem Außengewinde versehen ist,

Fig. 7    einen Längsschnitt durch den Schieber der Fig. 6, gesehen längs der Linie VII-VII der Fig. 8,

Fig. 8    eine Ansicht, gesehen in Richtung des Pfeiles VIII der Fig. 6,

Fig. 9    einen Längsschnitt durch ein teilweise montiertes Injektionsgerät nach der Erfindung in seinem Zustand nach einer Injektion bzw. vor der Einstellung einer Injektionsdosis,

Fig. 10    einen Längsschnitt durch eine Anordnung gemäß Fig. 9, aber nach der Einstellung einer Injektionsdosis,

Fig. 11    eine Seitenansicht, gesehen in Richtung des Pfeiles XI der Fig. 9, wobei das Injektionsgerät in der Stellung für die Dosis Null ist,

Fig. 12    eine Seitenansicht analog Fig. 11, wobei sich jedoch das Injektionsgerät in einer Stellung für die Dosis 40 Einheiten befindet,

Fig. 13    eine Draufsicht auf eine mit einem Außengewinde (Linksgewinde) versehene Kolbenstange, die dazu dient, Injektionsflüssigkeit aus einem Behälter (Kartusche) auszupressen, gesehen in Richtung des Pfeiles XIII der Fig. 14,

Fig. 14    einen Schnitt durch die Kolbenstange, gesehen längs der Linie XIV-XIV der Fig. 13,

Fig. 15    eine Draufsicht auf ein Gewindeteil, das auch als Zustellteil bezeichnet wird,

Fig. 16    einen Längsschnitt durch das Gewindeteil der Fig. 15, gesehen längs der Linie XVI-XVI der Fig. 17,

Fig. 17    einen Schnitt, gesehen längs der Linie XVII-XVII der Fig. 15,

Fig. 18    eine Draufsicht auf einen Mitnehmer, welcher dazu dient, das Gewindeteil (Fig. 5 bis 17) bei bestimmten Betriebszuständen mit dem Einstellglied (Fig. 4 und 5) so zu kuppeln, dass es sich mit diesem dreht, aber axial relativ zum Einstellglied frei verschiebbar ist,

Fig. 19    einen Längsschnitt durch den Mitnehmer der Fig. 18, gesehen längs der Linie XIX-XIX der Fig. 20,

Fig. 20    einen Schnitt, gesehen längs der Linie XX-XX der Fig. 18,

Fig. 21    einen Schnitt durch ein Führungsglied, das zur axialen Führung der Kolbenstange im Gehäuse dient, gesehen längs der Linie XXI-XXI der Fig. 22,

Fig. 22    eine Ansicht des Führungsglieds der Fig. 21, gesehen in Richtung des Pfeiles XXII der Fig. 21,

Fig. 23    einen schematischen Längsschnitt durch ein Injektionsgerät im montierten Zustand und vor der Einstellung einer Injektionsdosis,

Fig. 24    einen Schnitt, gesehen längs der Linie XXIV-XXIV der Fig. 23,

Fig. 25    einen Schnitt, gesehen längs der Linie XXV-XXV der Fig. 23,

Fig. 26    einen Schnitt, gesehen längs der Linie XXVI-XXVI der Fig. 23,

Fig. 27    einen Längsschnitt analog Fig. 23, aber nach der Einstellung einer Injektionsdosis,

Fig. 28    einen Längsschnitt analog Fig. 23 und 27, aber während einer Injektion,

Fig. 29    eine Variante zu dem Injektionsgerät nach den Figuren 1 bis 28, wobei der Injektionsvorgang durch eine Energie unterstützt wird, welche vom Benutzer während der Dosiseinstellung im Gerät gespeichert wird,

Fig. 30    eine zweite Variante, welche ebenfalls die Servounterstützung der Fig. 29 verwendet, wobei aber Maßnahmen getroffen sind, damit sich die Länge der verwendeten Torsionsfeder 148 im Betrieb nicht verändert,

Fig. 31    einen Schnitt, gesehen längs der Linie XXXI-XXXI der Fig. 30, und

Fig. 32    eine Gesamtdarstellung des injektionsgeräts.

Die nachfolgende Beschreibung erläutert zuerst an stark vergrößerten und schematisierten Darstellungen den prinzipiellen Aufbau und die Wirkungsweise der Erfindung, wobei die vorgenannte Einschränkung gilt, dass die Ausführungsbeispiele nicht patentgemäß sind, weil die erste Antriebsverbindung nicht durch ein zweites Gewinde, sondern durch die axiale Führung und die zweite Antriebsverbindung nicht durch eine axiale Führung, sondern durch das zweite Gewinde (86, 94) ausgebildet ist. Im Anschluss hieran folgt ein spezielles Ausführungsbeispiel in Form eines sogenannten Peninjektors. Dabei werden in der Beschreibung für gleiche oder gleich wirkende Teile jeweils dieselben Bezugszeichen verwendet, und diese Teile werden gewöhnlich nur einmal beschrieben.

Die Bewegungsrichtungen werden in der in der Medizin üblichen Weise angegeben, also

proximal = zum Patienten hin, also in Richtung zur Injektionsnadel

distal = vom Patienten weg, also in Richtung weg von der Injektionsnadel.

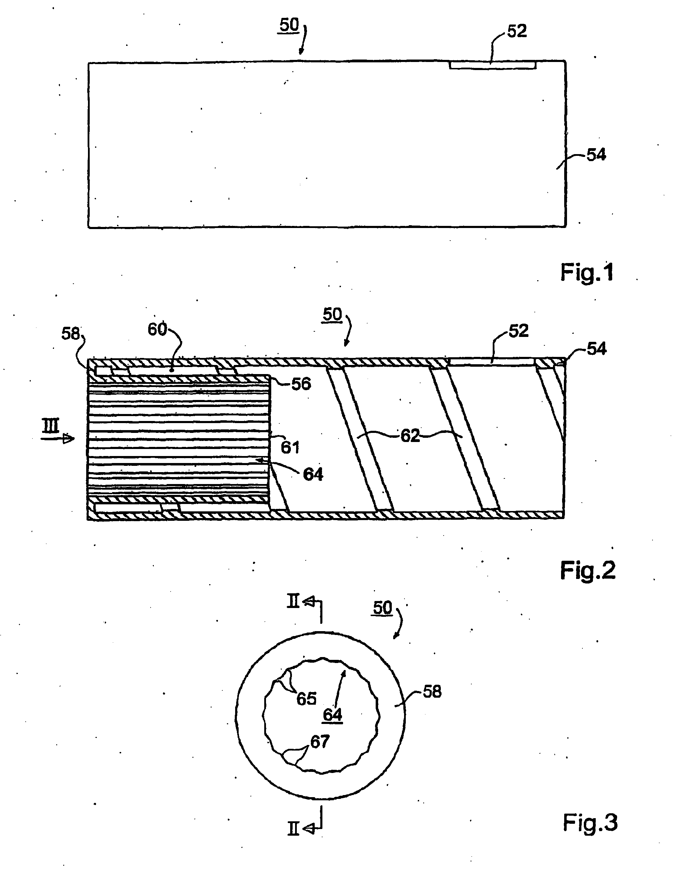

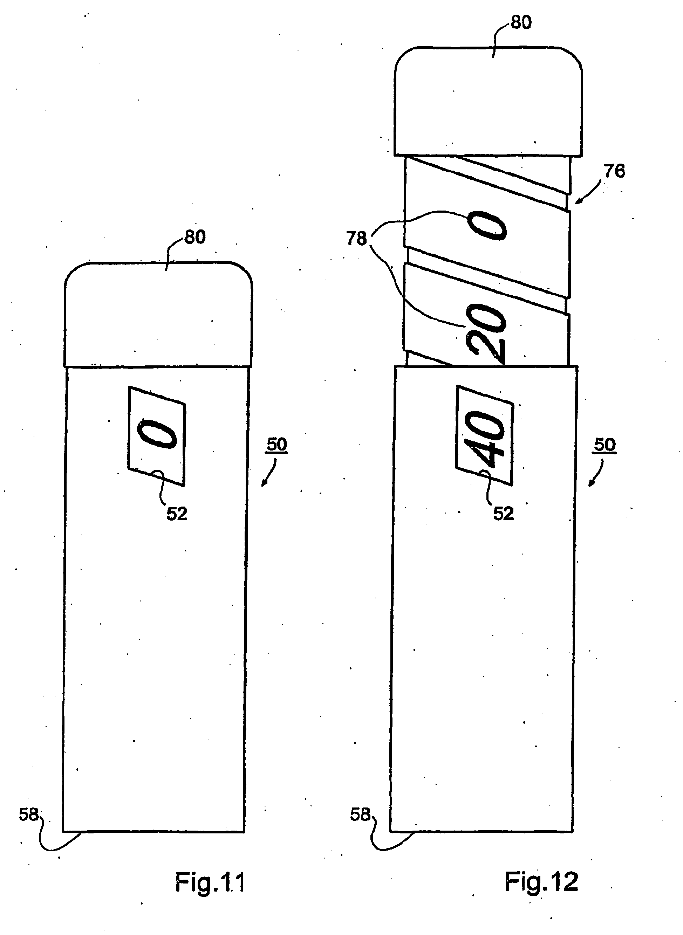

**Fig. 1** zeigt die Seitenansicht eines Gehäuses 50, das eine zylindrische Außenseite mit einem Fenster 52 hat, das zur (mechanischen) Anzeige der Injektionsdosis dient, vgl. Fig. 12, wo eine Anzeige von 40 Einheiten als Beispiel dargestellt ist.

Das Gehäuse 50 aus einem geeigneten Kunststoff hat ein Außenrohr 54 und ein dazu konzentrisches Innenrohr 56, die durch ein Brückenteil 58 (**Fig. 2 und 3**) so miteinander verbunden sind, dass zwischen ihnen ein Ringraum 60 gebildet wird. Das Innenrohr 56 hat eine Länge, die beim Ausführungsbeispiel etwa 4/10 der Länge des Außenrohres 54 beträgt. Sein distales Ende ist mit 61 bezeichnet.

Im Außenrohr 54 ist ein Innengewinde 62 ausgebildet, das beim Ausführungsbeispiel als Steilgewinde mit etwa rechteckförmigem Querschnitt der Gewindegänge ausgebildet ist, hier beispielhaft als Linksgewinde mit einer Steigung von 10 mm pro Umdrehung (die Darstellungen sind aus Gründen der Anschaulichkeit vergrößert).

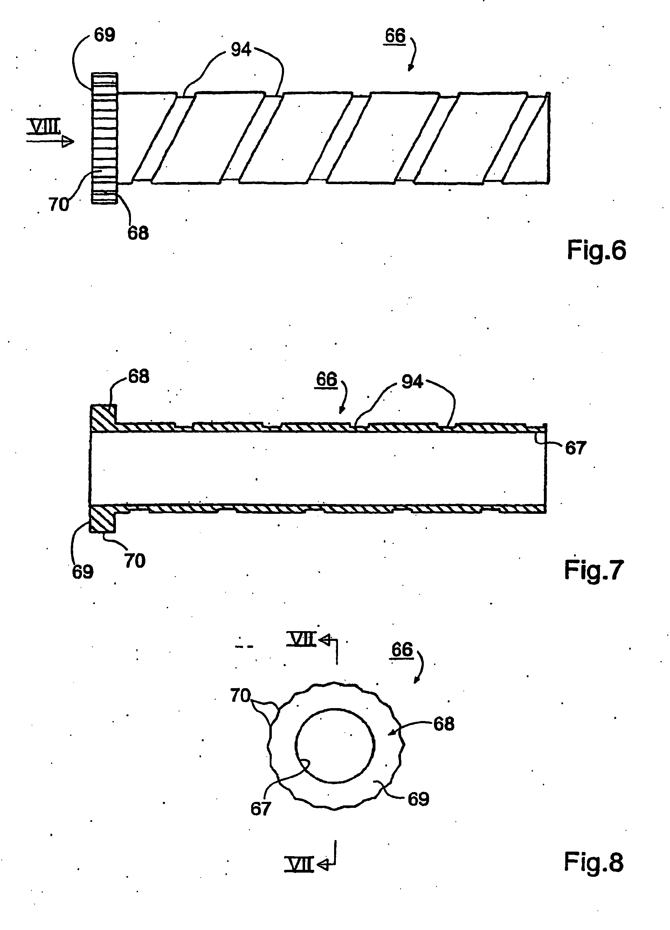

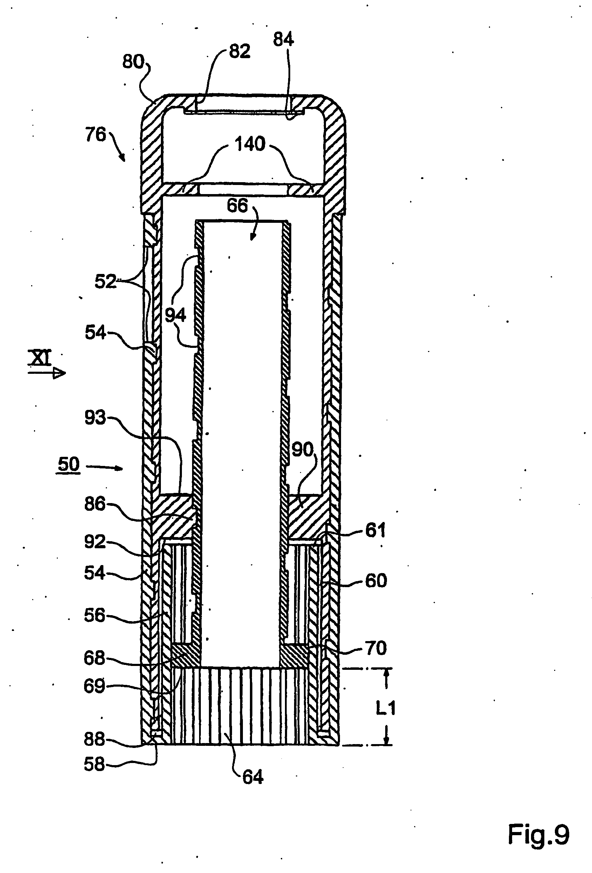

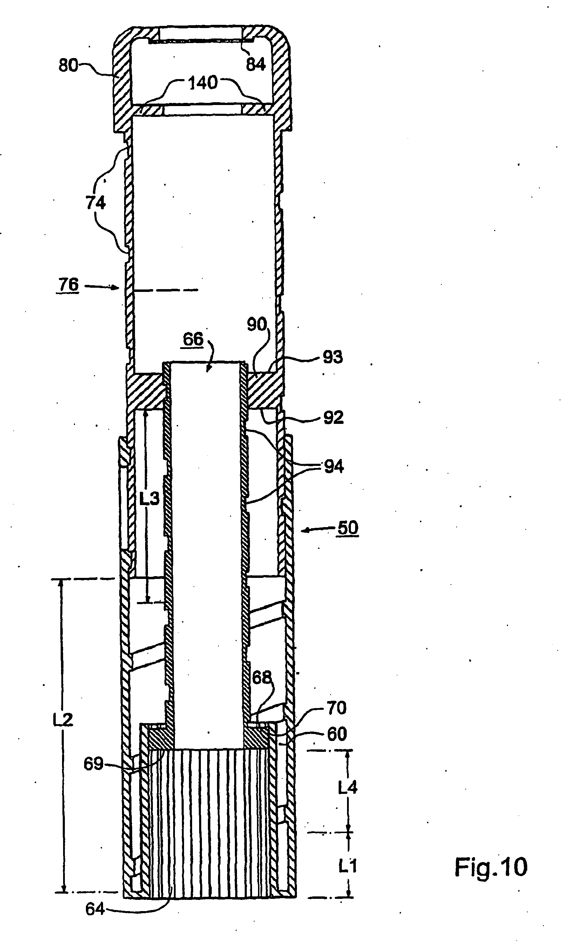

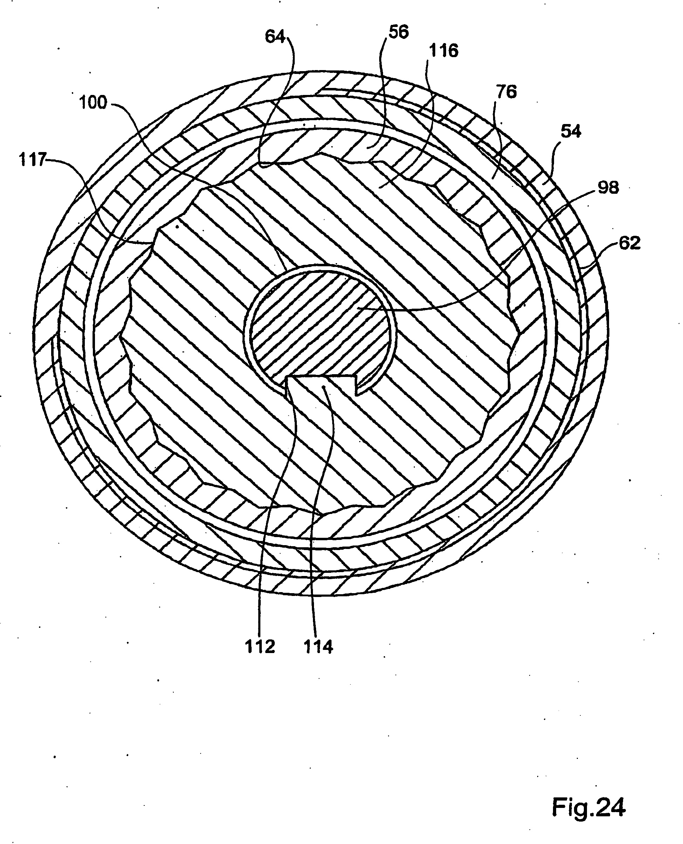

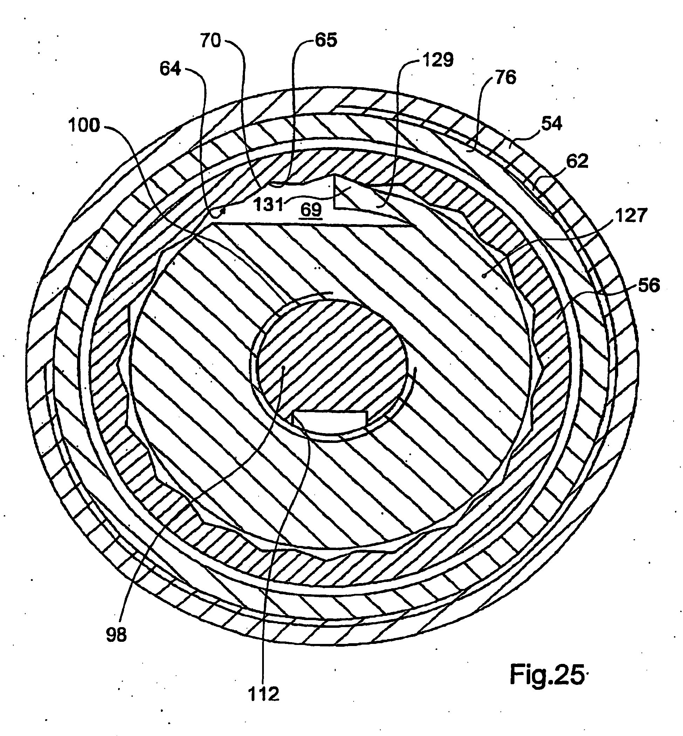

Im Innenrohr 56 ist bei dieser Ausführungsform eine Axialverzahnung 64 vorgesehen, deren Form aus den Fig. 2 und 3 hervorgeht. Sie hat hier zwanzig Längsnuten 65, zwischen denen sich Erhöhungen 67 befinden. Die Fig. 24 und 25 zeigen die Axialverzahnung 64 in stark vergrößertem Maßstab. Sie dient zur axialen Führung eines Schiebers 66, der in den Fig. 6 bis 8 dargestellt ist. Dieser hat an seinem proximalen Ende einen Kopfabschnitt 68 mit vergrößertem Durchmesser, und an diesem ist eine Axialverzahnung 70 vorgesehen, die zur Axialverzahnung 64 komplementär und in dieser geführt ist, vgl. z.B. Fig. 9 und 10. Das proximale Ende des Kopfabschnitts 68 ist mit 69 bezeichnet.

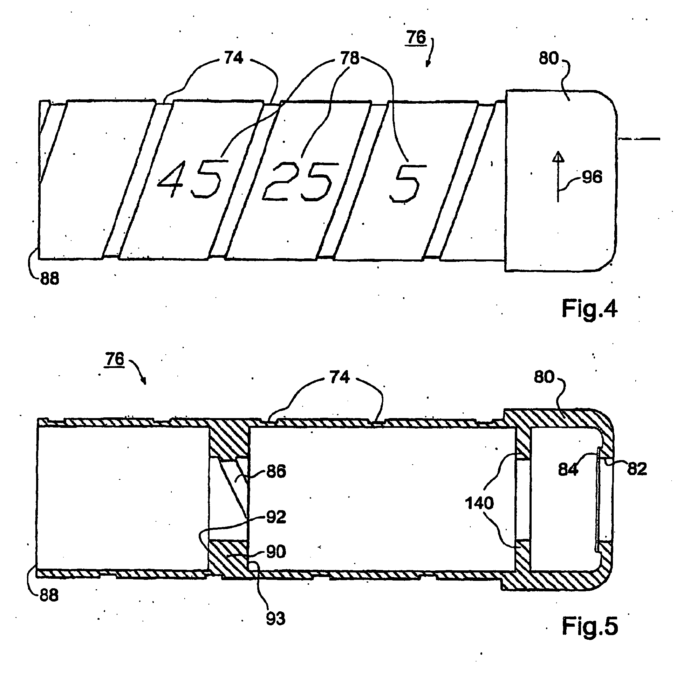

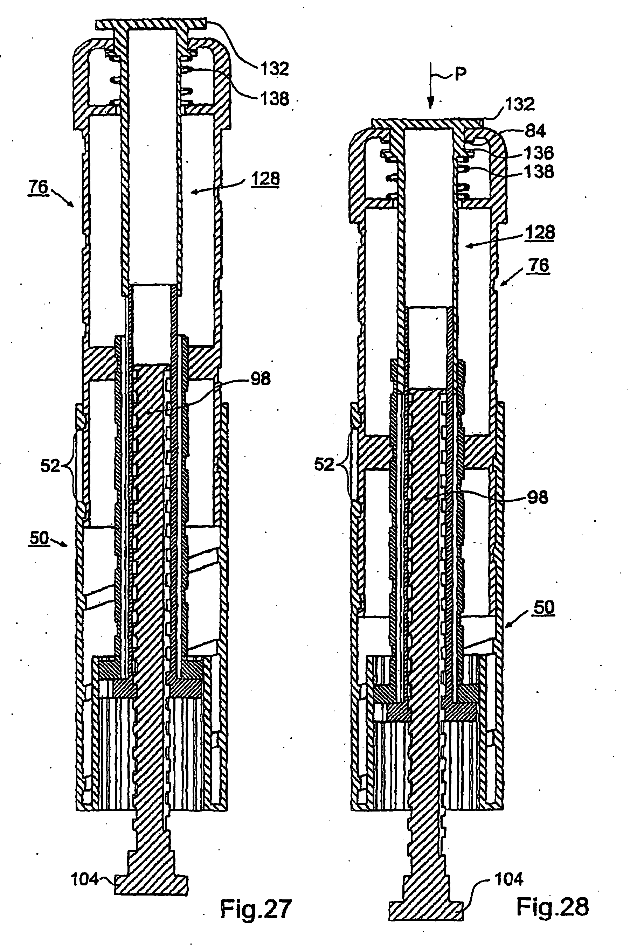

In dem Innengewinde 62 (Fig. 2) ist das Außengewinde 74 eines Einstellglieds 76 (**Fig. 4 und 5**) geführt. Letzteres hat zwischen seinen Gewindegängen Flächen, auf denen eine Dosisskala 78 angebracht ist, die z.B. von "0" bis "60" geht, weshalb man das Einstellglied 76 auch als Skalenrohr bezeichnen kann. Fig. 4 zeigt beispielhaft einige Zahlen der Skala. Das Glied 76 hat an seinem distalen Ende einen Einstellknopf 80, der zur Einstellung der Injektionsdosis dient und über den der Patient die eingestellte Dosis durch axialen Druck injiziert, vgl. nachfolgend Fig. 28. Im Knopf 80 ist eine zentrale Öffnung 82 vorgesehen, an deren Rand auf der proximalen Seite eine Verzahnung 84 ausgebildet ist.

Im Inneren des Einstellglieds 76 ist auf einem radial nach innen ragenden Gewindeträger 90 ein Innengewinde 86 ausgebildet, das hier als Links- und Steilgewinde mit einer Steigung von z.B. 7 mm pro Umdrehung ausgebildet ist. Seine Gewindegänge haben bevorzugt ebenfalls einen rechteckförmigen Querschnitt.

Wie aus **Fig. 9** hervorgeht, ist der Gewindeträger 90 in einem solchen Abstand vom proximalen Ende 88 des Einstellglieds 76 vorgesehen, dass letzteres voll in den Ringraum 60 (Fig. 2) hineingeschraubt werden kann, wobei der Gewindeträger 90 mit seiner proximalen Seite 92 zum Anschlag gegen das distale Ende 61 des Innenrohrs 56 gelangt. Die distale Seite des Gewindeträgers 90 ist mit 93 bezeichnet.

Die **Fig. 6 bis 8** zeigen den Schieber 66. Dieser hat ein Außengewinde (Linksgewinde) 94, das im montierten Zustand (Fig. 9 und 10) in das Innengewinde 86 des Einstellglieds 76 eingreift, so dass eine Drehung des Einstellglieds 76, bei der dieses in Richtung eines Pfeiles 96 (Fig. 4 gedreht wird, das Einstellglied 76 in distaler Richtung bewegt, während dieselbe Drehung den Schieber 66 relativ zum Einstellglied 76 in proximaler Richtung bewegt. Der Schieber 66 hat eine zylindrische Innenausnehmung 67, die in Fig. 7 links in die radial nach außen gehende Schulter 69 übergeht.

Fig. 9 zeigt die beschriebenen Teile vor der Einstellung einer Injektionsdosis, wobei gemäß Fig. 11 im Fenster 52 die Dosis "0" angezeigt wird. Das proximale Ende 69 des Schiebers 66 hat hier einen Abstand L1 vom proximalen Ende des Brückenteils 58.

Fig. 10 zeigt die Teile nach der Einstellung einer großen Injektionsdosis, nämlich nach drei vollen Umdrehungen des Einstellglieds 76. Dieses hat sich dadurch in distaler Richtung verschoben, z.B. um 30 mm. Gleichzeitig hat sich der Schieber 66 relativ zum Gewindeträger 90 um einen Abstand L3 in proximaler Richtung verschoben, im Beispiel um 21 mm. Die Wirkung ist, dass sich das proximale Ende 69 des Schiebers 66 gegenüber Fig. 9 um einen Weg

$$L4 = L2 - L3 \qquad \ldots (1)$$

in distaler Richtung verschoben hat, hier also um

$$30 - 21 = 9 \text{ mm} \qquad \ldots (2).$$

Diese 9 mm sind der Weg, der anschließend die eingespritzte Injektionsdosis bestimmt. Dies ist die Folge davon, dass der Schieber 66 durch seine Axialverzahnung 70 (Fig. 6 und 8) in der Axialverzahnung 64 des Gehäuses 50 axial geführt ist.

Das erfindungsgemäße Ausrührungsbeispiel erzielt hinsichtlich des Schiebers (66) die gleiche Wirkung wie die vorgenannten nicht patentgemäßen Ausführungsbeispiele, indem der Schieber (66) im Teil (90) axial geführt und durch ein Innengewinde im inneren Rohr (56) angetrieben wird. Es erfolgt also erfindungsgemäß gegenüber den dargestellten Ausführungsformen eine "kinematische Umkehr", das heißt die beiden Antriebsverbindungen für den Schieber (66) sind erfindungsgemäß ausgetauscht. Das Gewinde wird also bei einer erfindungsgemäßen Ausführungsform zwischen der Außenseite des Teils (68) und der Innenseite des inneren Rohrs (56) angeordnet, also anstelle der axialen Führung (64, 76). Da jedoch die Axialverzahnung (64) des dargestellten nicht patentgemäßen Ausführungsbeispiels auch die Funktion einer Ratsche hat, welche bei der Dosiseinstellung wirksam wird, wird im Folgenden weiter dieses nicht patentgemäße Beispiel erörtert, welches eine "kinematische Umkehrung" des Erfindungsgegenstandes darstellt.

Durch die Dosiseinstellung erzeugt man also gegenläufige Bewegungen, d.h. das Einstellglied 76 bewegt sich schnell in distaler Richtung, und der Schieber 66 bewegt sich gleichzeitig etwas langsamer relativ zum Einstellglied 76 in proximaler Richtung, und als Endresultat verschiebt sich das proximale Ende 69 des Schiebers 66 um einen relativ kleinen Weg L4 in distaler Richtung. Die Anordnung gemäß Fig. 9 und 10 wirkt also wie ein lineares Getriebe, und die große Bewegung des Einstellglieds 76 hat den Vorteil, dass eine Dosisanzeige (im Fenster 52) mit großen, gut lesbaren Ziffern 78 möglich ist, vgl. die Fig. 11 und 12. Auch kann eine Dosis, die versehentlich zu groß eingestellt wurde, manuell durch Drehen am Einstellknopf 80 korrigiert werden, wobei sich dann der Weg L4 wieder verkleinert. Zudem kann der Patient während der Injektion im Fenster 52 genau verfolgen, wieviel er bereits injiziert hat. Viele Patienten wollen diese Information haben.

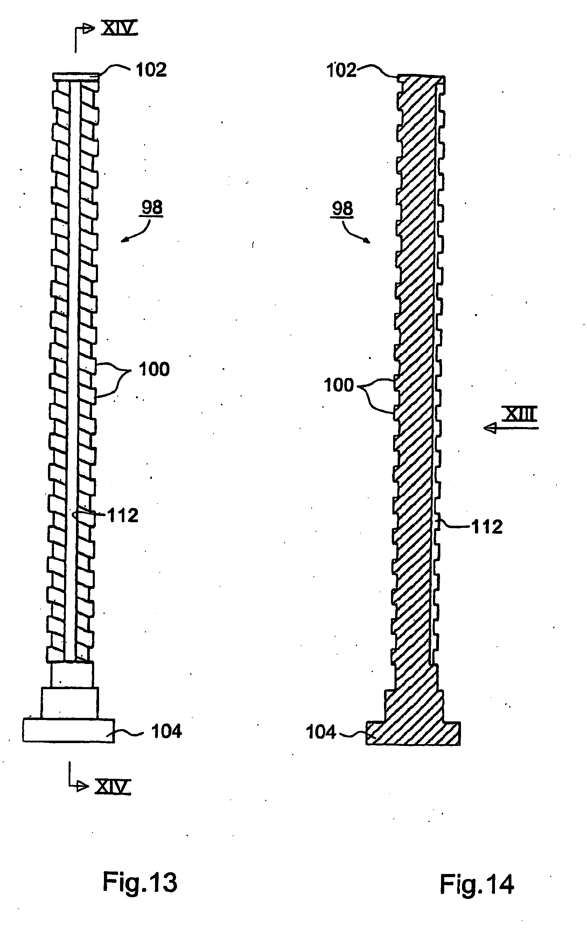

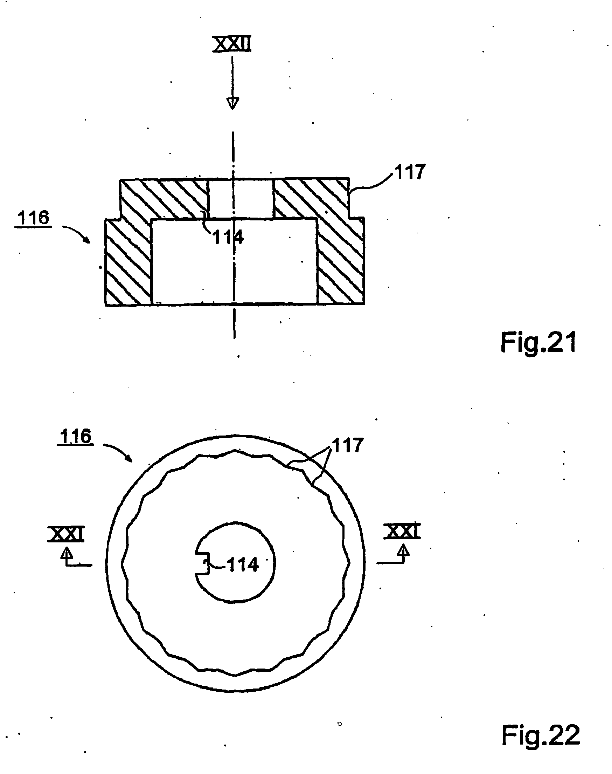

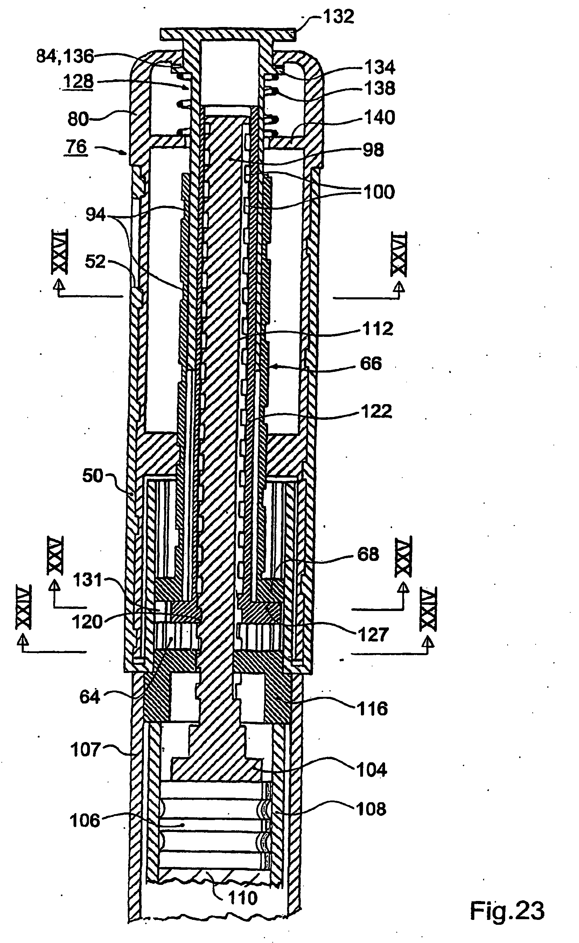

Die **Fig. 13 und 14** zeigen eine Gewindestange 98, die mit einem Rechteck-Steilgewinde 100 versehen ist, das, wie dargestellt, ein Linksgewinde ist und dessen Gewindesteigung bei diesem Beispiel 3 mm pro Umdrehung beträgt. Die Kolbenstange 98 hat an ihrem distalen Ende einen Anschlag 102, der ein vollständiges Herausschrauben verhindert, und an ihrem proximalen Ende, also in den Fig. 13 und 14 unten, hat sie eine Schubplatte 104, mit der sie im montierten Zustand gemäß Fig. 23 gegen den Gummikolben 106 einer Kartusche 108 anliegt, die mit Injektionsflüssigkeit 110 gefüllt ist. Ferner hat sie eine Längsnut 112, mit der sie in einem Teil 116 axial geführt ist, das in den **Fig. 21 und 22** dargestellt ist und das mit einem Vorsprung 114 in die Längsnut 112 eingreift. Das Teil 116 hat auf seiner Außenseite einen Abschnitt mit einer Axialverzahnung 117, mit dem es im montierten Zustand in der Axialverzahnung 64 des Gehäuses 50 geführt ist. Dadurch entsteht eine axiale Führung 112, 114 der Kolbenstange 98 relativ zum Gehäuse 50.

Das Teil 116 gemäß Fig. 21 und 22 steckt mit seiner Außenverzahnung 117 in der Axialverzahnung 64 des Gehäuses 50 und wird im montierten Zustand in dieser Lage festgehalten, weil es gemäß Fig. 23 mit seinem proximalen Ende gegen das distale Ende der Kartusche 108 anliegt.

Wird das Gerät geöffnet, indem in Fig. 23 ein proximales Gehäuseteil 107 vom Gehäuseteil 50 abgenommen wird, so wird das Teil 116 nicht mehr von der Kartusche 108 abgestützt und kann bis zu einem Anschlag aus der Axialverzahnung 64 herausgezogen werden. Dadurch wird es frei drehbar und ermöglicht es, die Kolbenstange 98 in distaler Richtung durch Drehung relativ zum Gewinde 98 wieder in ihre Anfangsstellung zurück zu schrauben. Dies ermöglicht das Laden einer neuen, gefüllten Kartusche 108.

Bei einer Injektion verschiebt in Fig. 23 die Kolbenstange 98 den Kolben 106 in proximaler Richtung, also nach unten, und presst dadurch Injektionsflüssigkeit 110 aus dem Behälter 108. Letzterer kann ausgewechselt werden, wenn die Flüssigkeit 110 verbraucht ist. Das Gerät wird gewöhnlich leer geliefert, also ohne Behälter (Kartusche) 108. Bei der Injektion führt die Kolbenstange 98 keine Drehbewegung aus, sondern bewegt sich linear in proximaler Richtung.

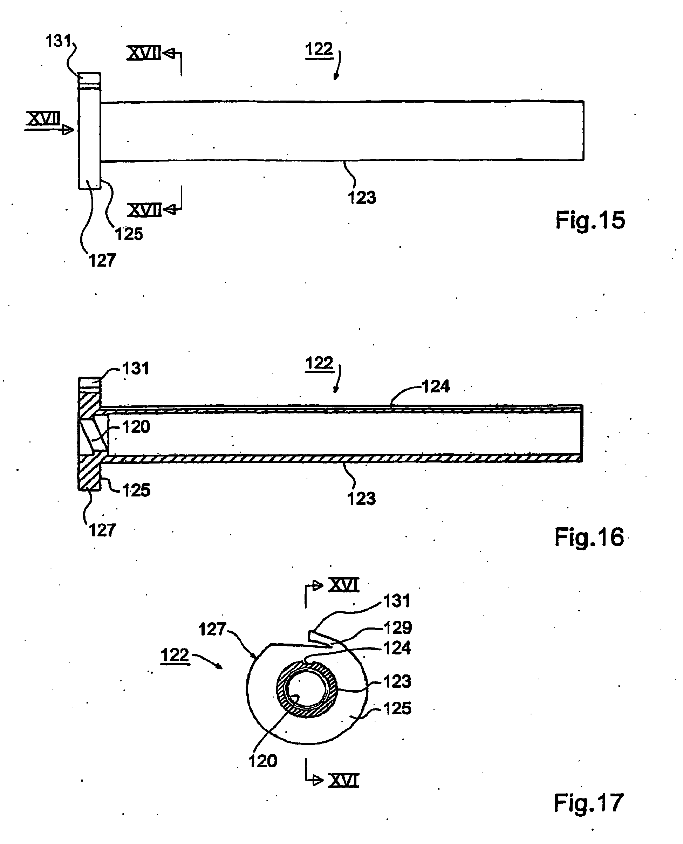

Das Außengewinde 100 der Kolbenstange 98 ist in einem Innengewinde 120 eines Gewindeteils 122 (Fig. 15 bis 17) geführt, das im Folgenden auch als Zustellteil bezeichnet wird. Wenn das Gewindeteil 122 gedreht wird, bewirkt es eine axiale Verschiebung der Kolbenstange 98 relativ zu diesem Teil 122. Diesen Vorgang bezeichnet man als Zustellung (Einstellung) der Kolbenstange 98, und daher der Name "Zustellteil".

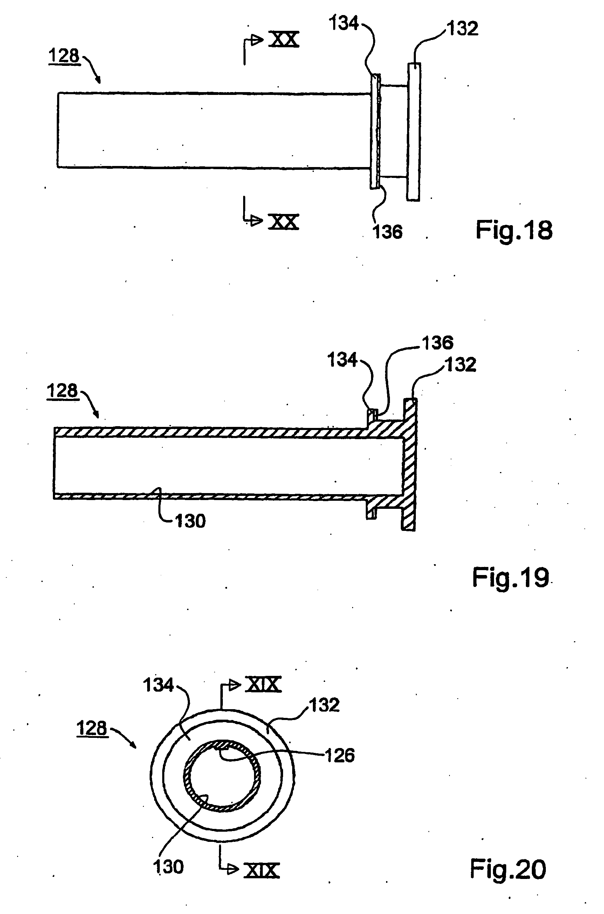

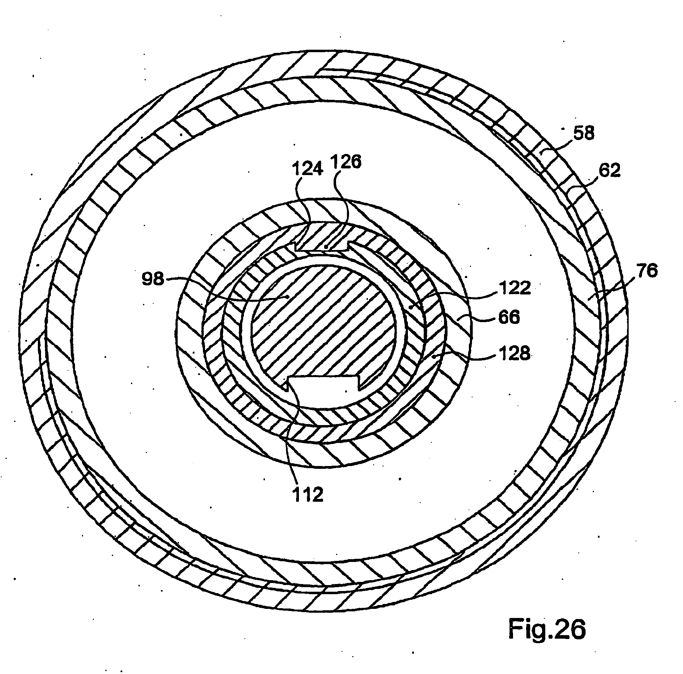

Das Teil 122 hat auf seiner zylindrisch ausgebildeten Außenseite 123 eine Längsnut 124. In diese greift ein radial nach innen ragender Vorsprung 126 (Fig. 20) eines Mitnehmers 128 (Fig. 18 bis 20) ein. Letzterer hat eine zylindrische Innenausnehmung 130, die auf der zylindrischen Außenseite 123 des Gewindeteils 122 gleitend verschiebbar ist, wobei der Vorsprung 126 in der Längsnut 124 gleitet und die Teile 122, 128 drehfest miteinander verbindet, vgl. Fig. 23 und 26. Die zylindrische Außenseite 123 geht links in eine radial nach außen verlaufende Schulter 125 über, vgl. Fig. 15 bis 17.

Das Zustellglied 122 hat auf seiner proximalen Seite ein Kopfteil 127 aus einem elastischen Kunststoff. Das Kopfteil 127 ist einstückig mit einer radial federnden Rastzunge 129, an deren freiem Ende sich ein Rastglied 131 befindet, das gemäß Fig. 25 mit Vorspannung gegen die axiale Innenverzahnung 64 des Gehäuses 50 anliegt und in die Längsnuten 65 dieser Axialverzahnung 64 einrasten kann.

Bei einer Injektion verschiebt sich das Kopfteil 127 mit seinem Rastglied 131 axial in der Axialverzahnung 64.

Das Zusammenwirken von Rastglied 131 und Axialverzahnung 64 bewirkt, dass der Patient pro Umdrehung des Einstellglieds 76 (Fig. 4 und 5) zwanzig Clicks hört und spürt, so dass er/sie die Dosis auch nach Gehör oder Gespür einstellen kann, weil pro Einheit ein hör- und spürbares Signal erzeugt wird. Dies ist wichtig, weil viele Diabetiker sehschwach sind.

Zudem kann eine einmal eingestellte Dosis nicht unabsichtlich verstellt werden, da für eine Verstellung in beiden Drehrichtungen ein vorgegebenes Mindest-Drehmoment erforderlich ist.

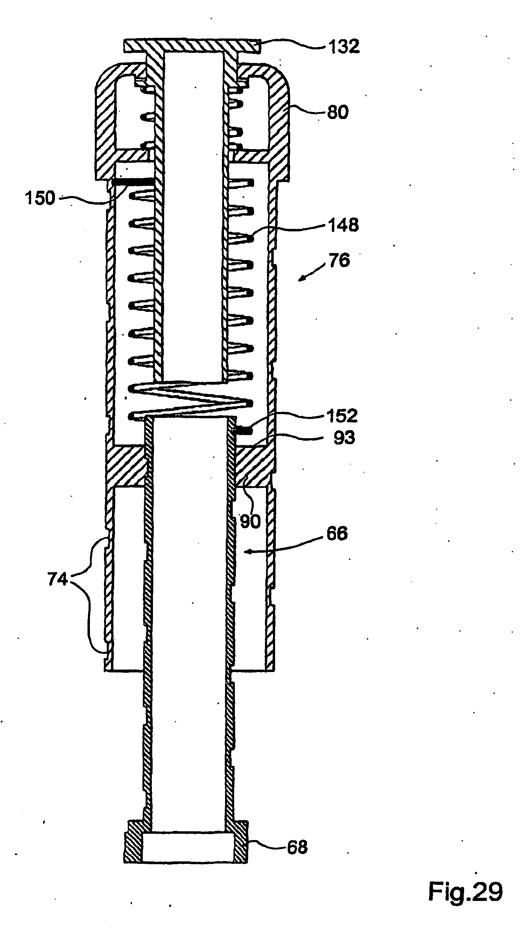

Schließlich bewirken Rastglied 131 und Rastverzahnung 64 in ihrem Zusammenwirken auch, dass eine Torsionsfeder 148 gemäß Fig. 29 bis 31 erst wirksam werden kann, wenn der Patient gemäß Fig. 28 auf den Mitnehmer 128 drückt und dadurch die Kupplung 84, 136 öffnet, da dann die Verbindung vom Rastglied 131 zum Einstellglied 76 unterbrochen wird und folglich die Torsionsfeder 148 das Einstellglied 76 verdrehen oder zumindest die Verdrehung des Einstellglieds 76 unterstützen kann.

Die Axialverzahnung 64 hat also im Rahmen des Ausführungsbeispiels mehrere Funktionen, denn sie dient zur drehfesten Verbindung zwischen Gehäuse 50 und Teilen, die mit diesem drehfest verbunden werden müssen, und auch zur Herstellung einer Rastverbindung, deren Funktion unabhängig von der axialen Stellung des Kopfteils 127 ist.

Der Mitnehmer 128 (Fig. 18 bis 20) hat an seinem distalen Ende eine Betätigungsplatte 132, auf die der Patient bei der Injektion in proximaler Richtung drückt, vgl. Fig. 28. Im Abstand von der Platte 132 ist ein Kupplungsflansch 134 angeordnet, der auf seiner distalen Seite, also in Fig. 18 und 19 rechts, mit einer Verzahnung 136 versehen ist, die zum Eingriff mit der in Fig. 5 dargestellten Verzahnung 84 dient und bei Eingriff die Teile 76 und 128 drehfest miteinander kuppelt.

Gemäß **Fig. 23** wird der Kupplungsflansch 134 durch eine Druckfeder 138 in distaler Richtung beaufschlagt. Die Feder 138 ist zwischen einem Anlageflansch 140 des Einstellglieds 76 und dem Kupplungsflansch 134 angeordnet, so dass die Verzahnungen 84, 136 in Eingriff miteinander stehen, solange der Patient nicht auf die Betätigungsplatte 132 drückt. Tut er das, so wird die Feder 138 zusammengedrückt, und die Verzahnungen 84, 136 werden außer Eingriff gebracht.

Bei der Einstellung einer Injektionsdosis verdreht der Patient das Einstellglied 76 relativ zum Gehäuse 50, und bei dieser Drehung wird über die (geschlossene) Kupplung 84, 136 auch der Mitnehmer 128 um den gleichen Drehwinkel relativ zum Gehäuse 50 verdreht. Weil sich der Mitnehmer 128 dreht, dreht sich - über die Führung 124, 128 - auch das Gewindeteil 122 relativ zum Gehäuse 50, wobei die beschriebene Rastverbindung 64, 131 betätigt wird.

Weil sich einerseits das Gewindeteil 122 relativ zum Gehäuse 50 dreht, sich andererseits aber die Kolbenstange 98 nicht relativ zum Gehäuse 50 drehen kann, weil sie gemäß Fig. 24 durch den Vorsprung 114 axial geführt wird, bewegt sich die Kolbenstange 98 relativ zum Gewindeteil 122 in proximaler Richtung, ändert dabei aber aus den nachstehend angegebenen Gründen ihre Lage relativ zum Gehäuse 50 nicht.

Weil sich das Einstellglied 76 relativ zum Gehäuse 50 dreht, der Schieber 66 aber durch die Führung 64, 70 (Fig. 9) relativ zum Gehäuse 50 nicht verdrehbar ist, wird der Schieber 66 durch das Gewinde 86, 94 relativ zum Gehäuse 50 in distaler Richtung verschoben. Dabei ist die distale Bewegung des Schiebers 66 relativ zum Gehäuse 50 bevorzugt gleich groß wie die proximale Bewegung der Kolbenstange 98 relativ zum Gewindeteil 122.

Eine Folge der proximalen Bewegung der Kolbenstange 98 relativ zum Gewindeteil 122 ist, dass das Gewindeteil 122 relativ zum Gehäuse 50 distal verschoben wird, wobei sich die Kolbenstange 98 relativ zum Gehäuse 50 nicht bewegt.

(Alternativ wäre es theoretisch auch möglich, dass die Kolbenstange 98 relativ zum Gehäuse 50 in proximaler Richtung verschoben wird, während sich das Gewindeteil 122 in axialer Richtung nicht bewegt, also stehen bleibt. Dies wird aber dadurch verhindert, dass das Gewindeteil 122 leicht eine Bewegung in distaler Richtung relativ zum Gehäuse 50 ausführen kann, während umgekehrt eine Bewegung der Kolbenstange 98 in proximaler Richtung durch die Reibung des Kolbens 106 (Fig. 23) im Behälter 108 stark behindert wird, so dass dieser Kolben 106 als Widerlager wirkt, welches eine Bewegung der Kolbenstange 98 relativ zum Gehäuse 50 beim Einstellvorgang verhindert.)

In Fig. 10 wurden die Verhältnisse erläutert, die beim Ausführungsbeispiel auftreten, wenn das Einstellglied 76 um drei Umdrehungen verdreht wurde.

Dieses hat sich dadurch um einen Weg L2 = 30 mm nach oben verschoben. Gleichzeitig hat sich der Schieber 66 um einen Weg L3 = 21 mm relativ zum Einstellglied 66 nach unten verschoben, so dass der Schieber 66 gemäß den Gleichungen (1) und (2) um einen Weg L4 = L2 - L3 = 30 - 21 = 9 mm nach oben gewandert ist.

Bei drei vollen Umdrehungen des Teils 76 führt auch das Gewindeteil 122 (Fig. 15 bis 17) drei volle Umdrehungen aus, wodurch die Kolbenstange 98 in Fig. 23 um 9 mm nach unten verschoben wird, also genau um den Weg L4.

In der Praxis bedeutet das, dass in Fig. 23 bei einer Einstellbewegung des Einstellglieds 76 (zwecks Dosiseinstellung) die Lage der Anpressplatte 104 der Kolbenstange 98 relativ zum Gummikolben 106 unverändert bleibt, d.h. die Kolbenstange 98 behält beim Einstellvorgang ihre Lage relativ zum Gehäuse 50 unverändert bei. Erst bei der Injektion tritt eine Änderung dieser Lage ein. Dies ist eine Folge davon, dass Richtungen und Steigungen der beschriebenen drei Gewinde in einem vorgegebenen Verhältnis zueinander stehen, und dieses Verhältnis kann entsprechend den Erfordernissen gewählt werden.

**Fig. 27** ist eine erweiterte Darstellung analog Fig. 10, und aus ihr erkennt man, dass die Lage der Kolbenstange 98 im Vergleich zu Fig. 23 trotz des Einstellvorgangs unverändert geblieben ist.

**Fig. 28** zeigt einen Zwischenzustand im Verlauf einer Injektion. Diese wird dadurch eingeleitet, dass der Patient mit einer Kraft P in proximaler Richtung auf die Platte 132 drückt. Dadurch wird die Feder 138 zusammen gepresst, und die Kupplung 84, 136 wird geöffnet.

Durch die Kraft P, ggf. vermehrt um das Drehmoment einer nachfolgend beschriebenen Torsionsfeder 148, wird das Einstellglied 76 in die Position gemäß Fig. 9 bzw. Fig. 23 zurück geschraubt, da es ein Steilgewinde hat, das bei axialem Druck automatisch eine Schraubbewegung durchführt. Mit dem Einstellglied 76 wird auch der Schieber 66 in die Position gemäß Fig. 9 bzw. Fig. 23 zurück geschraubt.

Die Lage der Kolbenstange 98 relativ zum Gewindeteil 122 bleibt während des Injektionsvorgangs unverändert, und da sich der Schieber 66 bei diesem Vorgang in Fig. 10 um den Weg L4 nach unten bewegt, wird das Gewindeteil 122 durch den Schieber 66 um den Weg L4 nach unten verschoben, also in proximaler Richtung, da die Schulter 69 des Schiebers 66 in proximaler Richtung gegen die Schulter 125 des Gewindeteils 122 drückt.

Durch das Gewindeteil 122 wird auch die Kolbenstange 98 um den Weg L4 nach unten verschoben und presst dabei den Gummikolben 106 (Fig. 23) um den Weg L4 nach unten, um eine entsprechende Menge Injektionsflüssigkeit 110 aus der Kartusche 108 heraus zu pressen. Man erreicht also, dass eine Menge an Injektionsflüssigkeit injiziert wird, die dem zuvor eingestellten Weg L4 entspricht.

Da sich das Einstellglied 76 beim Injektionsvorgang dreht, kann der Patient im Fenster 52 wie in einem Film den Ablauf der Injektion verfolgen, d.h. er weiß in jedem Augenblick, wieviel er bereits injiziert hat. Wenn im Skalenfenster 52 die Zahl 0 erscheint, weiß der Patient, dass er seine volle Dosis injiziert hat.

Am Ende einer Injektion erscheint also im Fenster 52 automatisch die Anzeige "0", vgl. Fig. 11, und ein neuer Einstellvorgang kann beginnen, so dass für den Patienten keine Rechenoperationen, Rückstellvorgänge oder dergleichen notwendig sind.

**Fig. 29** zeigt eine Variante, bei der zwischen dem Einstellglied 76 und dem Schieber 66 eine Torsionsfeder 148 angeordnet ist. Das distale Ende 150 der Feder 148 ist mit dem Einstellglied 76 drehfest verbunden, und das proximale Ende 152 ist mit dem distalen Ende des Schiebers 66 drehfest verbunden.

Vor einer Injektion verdreht der Patient das Einstellglied 76 und schraubt dieses dadurch aus dem Gehäuse 50 heraus, wie bei Fig. 9 und 10 beschrieben. Dabei verdrehen sich Einstellglied 76 und Schieber 66 relativ zueinander, wie das bei Fig. 9 und 10 beschrieben wurde. Diese Verdrehung spannt die Feder 148 auf Torsion, was dadurch unterstützt werden kann, dass die Feder 148 mit einer vorgegebenen Vorspannung eingebaut wird.

Bei einer Injektion wird die relative Verdrehung zwischen dem Einstellglied 76 und dem Schieber 66 wieder rückgängig gemacht, und das Injektionsgerät kehrt z.B. aus der Stellung gemäß Fig. 10 in die Stellung gemäß Fig. 9 zurück, wobei die eingestellte Dosis injiziert wird.

Hierbei muss die Reibung des Kolbens 106 (Fig. 23) im Behälter 108 überwunden werden, und dies wird durch die Energie erleichtert, welche bei der Dosiseinstellung in der Torsionsfeder 148 gespeichert wurde, so dass die Injektion für den Patienten erleichtert wird.

Wie ein Vergleich der Fig. 9 und 10 zeigt, würde die Feder 148 in Fig. 29 beim Einstellen einer Injektionsdosis nicht nur auf Torsion gespannt, sondern auch in die Länge gezogen, und umgekehrt würde sie bei einer Injektion stark zusammengepresst, wobei sich Platzprobleme ergeben könnten.

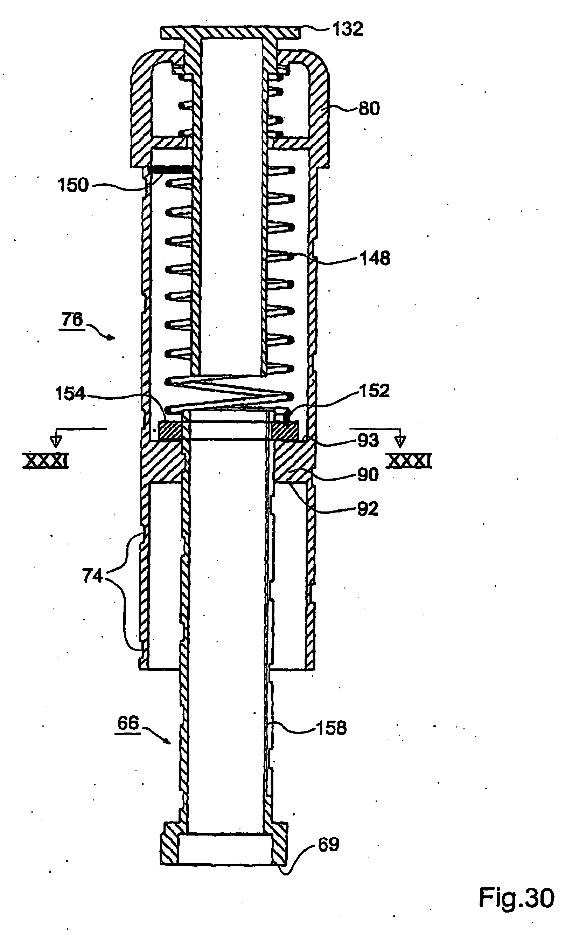

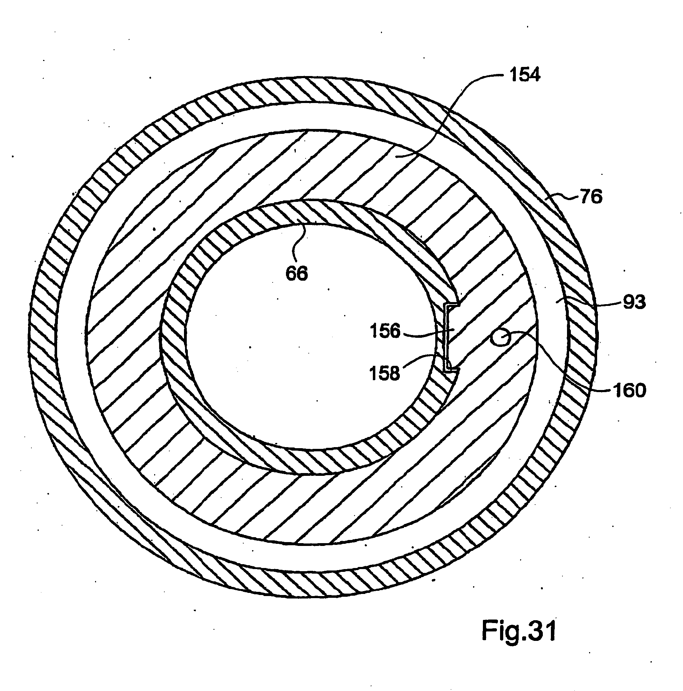

Diese Platzprobleme werden vermieden bei der Version nach **Fig. 30**. Dort ist ein Verschiebeglied 154 vorgesehen, welches auf der zylindrischen Außenfläche des Schiebers 66 gleitet. Das Verschiebeglied 154 hat einen Vorsprung 156 (Fig. 31), der radial nach innen ragt, und der Schieber 66 ist mit einer Längsnut 158 versehen, in die dieser Vorsprung 156 eingreift. Das proximale Ende 158 der Feder 148 greift wie dargestellt in eine Ausnehmung 160 des Verschiebeglieds 154 ein.

Durch die Spannkraft der Feder 148 wird das Verschiebeglied 154 stets in Anlage gegen die distale Seite 93 des Gewindeträgers 90 gehalten, so dass sich bei der Dosiseinstellung und beim Injizieren bei der Variante nach Fig. 30 und 31 die Länge der Feder 158 nicht ändert. Bei der Drehung des Schiebers 66 führt auch das Verschiebeglied 154 eine entsprechende Drehung relativ zum Gewindeträger 90 aus, weshalb diese Teile aus einem Kunststoff mit niedrigem Reibungskoeffizienten hergestellt werden sollten, oder man kann zwischen diesen Teilen eine Unterlegscheibe aus PTFE oder dgl. vorsehen.

Wie bereits beschrieben, wird die in der Feder 148 gespeicherte Energie erst frei gegeben, wenn durch einen Druck auf die Platte 132 die Kupplung 84, 136 geöffnet wird, vgl. Fig. 28, da hierdurch die Verbindung zu dem in Fig. 25 dargestellten Rastglied 131 unterbrochen wird, welches vor einer Injektion die Feder 148 in ihrer gespannten Stellung fest hält, soweit die Rastkraft hierzu ausreicht.

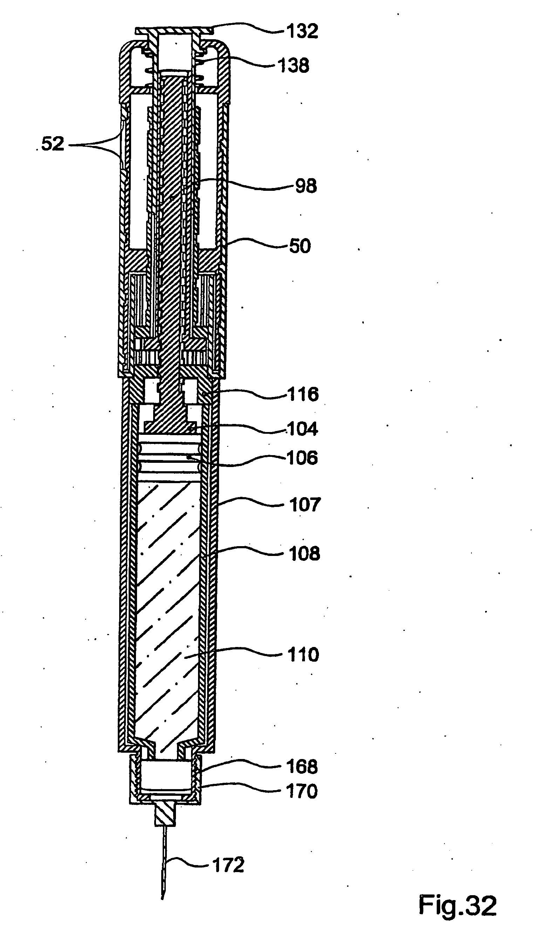

**Fig. 32** zeigt das Injektionsgerät in einer Gesamtdarstellung. Der obere, distale Teil entspricht der Darstellung gemäß Fig. 23, so dass hierauf verwiesen werden kann. Die Kartusche 108 ist im proximalen Gehäuseteil 107 geführt. Dieses hat unten ein Gewinde 168 zum Anschrauben des Gewindeteils 170 einer Kanüle 172, deren distaler Teil in bekannter Weise durch eine (nicht dargestellte) Gummimembran der Kartusche 108 durch sticht und dadurch eine Verbindung der Kanüle 172 mit der Injektionsflüssigkeit 110 in der Kartusche 108 herstellt, wie das dem Fachmann bekannt ist. Die Kanüle 172 wird gewöhnlich vor jeder Injektion ausgewechselt.

Naturgemäß sind im Rahmen der vorliegenden Erfindung vielfache Abwandlungen und Modifikationen möglich.

**Patentansprüche**

**1.** Injektionsgerät, welches aufweist:

Ein Gehäuse (50), welches zur Aufnahme eines Behälters (108) mit Injektionsflüssigkeit (110) ausgebildet ist; eine mit einem Gewinde (100) versehene Kolbenstange (98) zum Auspressen von Injektionsflüssigkeit (110) aus einem solchen Behälter (108), welche Kolbenstange (98) relativ zum Gehäuse (50) unverdrehbar und in axialer Richtung geführt (112, 114) ist;

ein Gewindeteil (122), das mit einem Gewinde (120) versehen ist, welches mit dem Gewinde (100) der Kolbenstange (98) in Eingriff steht,

welches Gewindeteil (122) so ausgebildet ist, dass seine axiale Lage relativ zum Gehäuse (50) veränderbar ist, wobei das Gewindeteil (122) zum Einstellen einer Injektionsdosis relativ zur Kolbenstange (98) und relativ zum Gehäuse (50) verdrehbar ist,

und

wobei das Gewindeteil (122) bei einem Injektionsvorgang relativ zur Kolbenstange (98) und relativ zum Gehäuse (50) unverdrehbar ist, und sich deshalb bei einem Injektionsvorgang relativ zum Gehäuse (50) in axialer Richtung und in Injektionsrichtung bewegt,

wobei das Gewindeteil (122) sich beim Einstellen relativ zur Kolbenstange (98) und relativ zum Gehäuse (50) in axialer Richtung bewegt,

und wobei im Gehäuse (50) ein Einstellglied (76) drehbar angeordnet ist, wobei Gehäuse (50) und Einstellglied (76) zusammen eine kombinierte Länge haben, deren Größe vor der Einstellung einer Injektionsdosis einer vorgegebenen Ausgangslänge entspricht, wobei zur Einstellung einer Injektionsdosis diese kombinierte Länge durch eine Relativdrehung zwischen Gehäuse (50) und Einstellglied (76) vergrößerbar ist, und während einer Injektion diese kombinierte Länge, ebenfalls durch eine Relativdrehung zwischen Gehäuse (50) und Einstellglied (76), wieder auf die vorgegebene Ausgangslänge zurückführbar ist., **dadurch gekennzeichnet, daß** ein Schieber (66) vorgesehen ist, der mit dem Gehäuse (50) über eine erste Antriebsverbindung und mit dem Einstellglied (76) über eine zweite Antriebsverbindung verbunden ist,

wobei die erste Antriebsverbindung als zweites Gewinde (86, 94) und die zweite Antriebsverbindung als axiale Führung (64, 70) ausgebildet ist, so dass sich der Schieber (66) relativ zum Gehäuse (50) in einer vorgegebenen Richtung bewegt, wenn das Einstellglied (76) durch eine Drehung relativ zum Gehäuse (50) ebenfalls in dieser vorgegebenen Richtung bewegt wird.

2. Injektionsgerät nach Anspruch 1, bei welchem ein Mitnehmer (128) vorgesehen ist, welcher mit dem Einstellglied (76) so gekoppelt ist, dass eine zur Dosiseinstellung erfolgende Drehbewegung des Einstellglieds (76) auf den Mitnehmer (128) übertragen wird (Fig. 27),

und dass eine bei einer Injektion (Fig. 28) erfolgende Drehbewegung des Einstellglieds (76) nicht auf den Mitnehmer (128) übertragen wird.

3. Injektionsgerät nach Anspruch 2, bei welchem das Gewindeteil (122) mit dem Mitnehmer (128) so verbunden ist, dass eine Rotationsbewegung des Mitnehmers (128) auf das Gewindeteil (122) übertragen wird, nicht jedoch eine axiale Verschiebung des Mitnehmers (128) relativ zum Gehäuse (50).

4. Injektionsgerät nach einem der Ansprüche 1 bis 3, bei welchem zwischen Gehäuse (50) und Einstellglied (76) zur Längenverstellung ein drittes Gewinde (62, 74) angeordnet ist.

5. Injektionsgerät nach Anspruch 4, bei welchem die Steigungen der drei Gewinde so aufeinander abgestimmt sind, dass während der Einstellung einer Injektionsdosis die Bewegung des Schiebers (66) in einer vorgegebenen Richtung mindestens gleich groß ist wie die Bewegung des Gewindeteils (122) in dieser Richtung.

6. Injektionsgerät nach Anspruch 4 oder 5, bei welchem Abschnitte des Einstellglieds (76), welche im Bereich des dritten Gewindes (52, 74) liegen, mit Skalenwerten (78) für die Anzeige der eingestellten Injektionsdosis versehen sind.

7. Injektionsgerät nach Anspruch 6, bei welchem das Gehäuse (50) mit einem Fenster (52) zur Anzeige mindestens eines der Skalenwerte (78) versehen ist.

8. Injektionsgerät nach einem der Ansprüche 4 bis 8, bei welchem das zweite Gewinde (86, 94) und das dritte Gewinde (62, 74) gleiche Gewinderichtungen aufweisen.

9. Injektionsgerät nach Anspruch 8, bei welchem die Gewindesteigung des dritten Gewindes (62, 74) dem Betrag nach größer ist als die Gewindesteigung des zweiten Gewindes (86, 94).

**10.** Injektionsgerät nach Anspruch 80 oder 9, bei welchem die Gewinderichtung des ersten Gewindes (100, 120) mit der des dritten Gewindes (62, 74) übereinstimmt.

**11.** Injektionsgerät nach Anspruch 10, bei welchem die Gewindesteigung des ersten Gewindes (100, 120) dem Betrag nach kleiner ist als die Gewindesteigung des dritten Gewindes (62, 74).

**12.** Injektionsgerät nach einem der Ansprüche 1 bis 6, bei welchem der Schieber (66) auf seiner proximalen Seite mit einer Anlage (69) versehen ist, welche zur Anlage gegen eine distale Seite (125) des Gewindeteils (122) ausgebildet ist, um bei einer Injektion die Kolbenstange (98) über das Gewindeteil (122) in proximaler Richtung zu verschieben und Injektionsflüssigkeit (110) aus dem Behälter (108) auszupressen.

**13.** Injektionsgerät nach Anspruch 3 oder 4, bei welchem eine steuerbare Kupplung (84, 136) vorgesehen ist, welche im geschlossenen Zustand die Übertragung eines Drehmoments vom Einstellglied (76) zum Mitnehmer (128) ermöglicht.

**14.** Injektionsgerät nach Anspruch 15, bei welchem die steuerbare Kupplung (84, 136) so ausgebildet ist, dass sie während der Einstellung einer Injektionsdosis die Rotationsbewegung des Einstellglieds (76) auf den Mitnehmer (128) überträgt.

**15.** Injektionsgerät nach Anspruch 15 oder 16, bei welchem die steuerbare Kupplung (84, 136) so ausgebildet ist, dass sie während einer Injektion eine Rotationsbewegung des Einstellglieds nicht auf den Mitnehmer (128) überträgt.

**16.** Injektionsgerät nach einem der Ansprüche 15 bis 17, bei welchem die steuerbare Kupplung (84; 136) so ausgebildet ist, dass sie durch einen zu Beginn einer Injektion erfolgenden Vorgang geöffnet wird.

**17.** Injektionsgerät nach einem der Ansprüche 15 bis 18, bei welchem die steuerbare Kupplung (84, 136) durch den Injektionsvorgang so steuerbar ist, dass sie nach Beendigung einer Injektion in den geschlossenen Zustand zurückkehrt und dass sie während der Dosiseinstellung geschlossen bleibt, so dass eine zur Dosiseinstellung erfolgende Drehung des Einstellglieds (76) durch die steuerbare Kupplung (84, 136) auf das Gewindeteil (122) übertragen wird.

**18.** Injektionsgerät nach einem, der Ansprüche 1 bis 6, bei welchem die Gewindesteigungen von erstem, zweitem und drittem Gewinde so bemessen sind, dass beim Einstellvorgang eine Drehung des Einstellglieds (76) im Wesentlichen ohne Einfluss auf die Lage der Kolbenstange (98) relativ zum Gehäuse (50) ist.

**19.** Injektionsgerät nach einem der vorhergehenden Ansprüche, bei welchem eine Torsionsfeder (148) vorgesehen und so angeordnet ist, dass bei einer Dosiselnstellung Energie in ihr gespeichert und bei einem Injektionsvorgang Energie aus ihr freigegeben wird.

**20.** Injektionsgerät nach Anspruch 19, bei welchem die Torsionsfeder (148) zwischen dem Einstellglied (76) und dem Schieber (66) angeordnet ist, wobei eine relative-Drehung zwischen diesen Teilen das von der Torsionsfeder (148) erzeugte Drehmoment verändert.

**21.** Injektionsgerät nach Anspruch 20, bei welchem die Torsionsfeder (148) mit dem Schieber (66) drehfest, aber axial verschiebbar, verbunden ist.


**Claims**

**1.** Injection device which comprises:

   a housing (50) which is designed to receive a container (108) with injection fluid (110);
   a piston rod (98) which is provided with a thread (100) to express injection fluid (110) from such a container (108), which piston rod (98) cannot be turned relative to the housing (50) and is guided in the axial direction (112, 114);
   a threaded part (122) which is provided with a thread (120) which is in engagement with the thread (100) of the piston rod (98), which threaded part (122) is designed so that its axial position relative to the housing (50) can be altered, the threaded part (122) being rotatable relative to the piston rod (98) and relative to the housing (50) to set an injection dose,
and the threaded part (122) being unable to turn relative to the piston rod (98) and relative to the housing (50) during an injection operation and therefore moving relative to the housing (50) in the axial direction and in the injection direction during an injection operation,
wherein the threaded part (122) moves relative to the piston rod (98) and relative to the housing (50) in the axial direction during setting,
and wherein a setting member (76) is arranged rotatably in the housing (50), the housing (50) and setting member (76) together having a combined length the magnitude of which before the setting of an injection dose corresponds to a predetermined starting length, this combined length being capable of being increased by a relative rotation between the housing (50) and setting member (76) to set an injection dose, and this combined length being capable of being returned to the predetermined starting length again, likewise by a relative rotation between the housing (50) and setting member (76) during an injection, **characterised in that** a slide (66) is provided which is connected to the housing (50) through a first drive connection and to the setting member (76) through a second drive connection,
the first drive connection being embodied as a second thread (86, 94) and the second drive connection being embodied as an axial guide (64, 70) so that the slide (66) moves relative to the housing (50) in a predetermined direction when the setting member (76) is also moved in this predetermined direction by a rotation relative to the housing (50).

2.  Injection device according to claim 1, in which an entrainment means (128) is provided which is coupled with the setting member (76) so that a turning movement of the setting member (76) to set the dose is transmitted to the entrainment means (128) (fig. 27),
    and so that a turning movement of the setting member (76) during an injection (fig. 28) is not transmitted to the entrainment means (128).

3.  Injection device according to claim 2, in which the threaded part (122) is connected to the entrainment means (128) so that a rotational movement of the entrainment means (128) is transmitted to the threaded part (122) but not an axial movement of the entrainment means (128) relative to the housing (50).

4.  Injection device according to one of claims 1 to 3, in which a third thread (62, 74) is arranged between the housing (50) and setting member (76) to adjust the length.

5.  Injection device according to claim 4, in which the pitches of the three threads are matched to one another so that during the setting of an injection dose the movement of the slide (66) in a predetermined direction is at least equal in magnitude to the movement of the threaded part (122) in this direction.

6.  Injection device according to claim 4 or 5, in which portions of the setting member (76) lying in the area of the third thread (52, 74) are provided with scale values (78) to display the set injection dose.

7.  Injection device according to claim 6, in which the housing (50) is provided with a window (52) to display at least one of the scale values (78).

8.  Injection device according to one of claims 4 to 6, in which the second thread (86, 94) and the third thread (62, 74) have the same thread directions.

9.  Injection device according to claim 8, in which the thread pitch of the third thread (62, 74) is greater in magnitude than the thread pitch of the second thread (86, 94).

10. Injection device according to claim 8 or 9, in which the thread direction of the first thread (100, 120) is the same as that of the third thread (62, 74).

11. Injection device according to claim 10, in which the thread pitch of the first thread (100, 120) is smaller in magnitude than the thread pitch of the third thread (62, 74).

12. Injection device according to one of claims 1 to 6, in which the slide (66) is provided on its proximal side with an abutment (69) which is designed for abutment against a distal side (125) of the threaded part (122) in order to move the piston rod (98) by means of threaded part (122) in the proximal direction and express injection fluid (110) out of the container (108) during an injection.

**13.** Injection device according to claim 3 or 4, in which a controllable coupling (84, 136) is provided which in the closed state allows transmission of a turning moment from the setting member (76) to the entrainment means (128).

**14.** Injection device according to claim 15, in which the controllable coupling (84, 136) is designed so that it transmits the rotational movement of the setting member (76) to the entrainment means (128) during the setting of an injection dose.

**15.** Injection device according to claim 15 or 16, in which the controllable coupling (84, 136) is designed so that it does not transmit a rotational movement of the setting member to the entrainment means (128) during an injection.

**16.** Injection device according to one of claims 15 to 17, in which the controllable coupling (84, 136) is designed so that it is opened by an operation taking place at the beginning of an injection.

**17.** Injection device according to one of claims 15 to 18, in which the controllable coupling (84, 136) is controllable by the injection operation so that it returns to the closed state after completion of an injection and remains closed during the setting of the dose so that a rotation of the setting member (76) to set the dose is transmitted to the threaded part (122) by the controllable coupling (84, 136).

**18.** Injection device according to one of claims 1 to 6, in which the thread pitches of the first, second and third thread are such that during the setting operation a rotation of the setting member (76) essentially has no influence on the position of the piston rod (98) relative to the housing (50).

**19.** Injection device according to one of the preceding claims, in which a torsion spring (148) is provided and arranged so that energy is stored in it during the setting of a dose and energy is released from it during an injection operation.

**20.** Injection device according to claim 19, in which the torsion spring (148) is arranged between the setting member (76) and the slide (66), and a relative rotation between these parts changes the turning moment produced by the torsion spring (148).

**21.** Injection device according to claim 20, in which the torsion spring (148) is connected to the slide (66) so as to be fixed rotationally but displaceable axially.

**Revendications**

**1.** Appareil d'injection comprenant :

un boîtier (50), réalisé de sorte à recevoir un récipient (108) renfermant un liquide d'injection (110) ;
une tige (98) de piston munie d'un filetage (100), pour exprimer du liquide d'injection (110) hors d'un tel récipient (108), laquelle tige (98) de piston ne peut pas tourner vis-à-vis du boîtier (50), et est guidée en direction axiale (112, 114) ;
une partie filetée (122), dotée d'un filetage (120) qui est en prise avec le filetage (100) de la tige (98) de piston, laquelle partie filetée (122) est réalisée de telle sorte que sa position axiale puisse être modifiée par rapport au boîtier (50),

la partie filetée (122) pouvant, en vue du réglage d'une dose d'injection, être animée d'une rotation vis-à-vis de la tige (98) de piston et vis-à-vis du boîtier (50),
et
la partie filetée (122) ne pouvant pas tourner vis-à-vis de la tige (98) de piston et vis-à-vis du boîtier (50), lors d'une opération d'injection, et se mouvant par conséquent, vis-à-vis dudit boîtier (50), en direction axiale et dans la direction d'injection, lors d'une opération d'injection,
la partie filetée (122) se mouvant en direction axiale, vis-à-vis de la tige (98) de piston et vis-à-vis du boîtier (50), lors du réglage et

un organe de réglage (76) étant logé à rotation dans le boîtier (50), ledit boîtier (50) et ledit organe de réglage (76) présentant, conjointement, une longueur combinée dont la grandeur correspond à une longueur initiale préétablie, préalablement au réglage d'une dose d'injection, sachant que, en vue du réglage d'une dose d'injection, cette longueur combinée peut être augmentée par une rotation relative entre le boîtier (50) et l'organe de réglage (76), cette longueur combinée pouvant être de nouveau ramenée à la longueur initiale préétablie, au cours d'une injection, également grâce à une rotation relative entre le boîtier (50) et l'organe de réglage (76), **caractérisé en ce qu'**une pièce coulissante (66) reliée au boîtier (50) par l'intermédiaire d'une première liaison d'entraînement, et à l'organe de réglage (76) par l'intermédiaire d'une seconde liaison d'entraînement est prévue, sachant que la première liaison d'entraînement est réalisé en tant que deuxième filetage (86, 94), et que la seconde liaison d'entraînement est réalisé comme un guidage axial (64, 70), de sorte que ladite pièce coulissante (66) est déplacée dans une direction préétablie, vis-à-vis du boîtier (50), lorsque ledit organe de réglage (76) est également déplacé dans cette direction préétablie, suite à une rotation vis-à-vis dudit boîtier (50).

2. Appareil d'injection selon la revendication 1, dans lequel est prévu un organe d'entraînement (128) accouplé, à l'organe de réglage (76), de façon telle qu'un mouvement rotatoire dudit organe de réglage (76), s'opérant en vue du réglage d'une dose, soit répercuté sur ledit organe d'entraînement (128) (figure 27) ; et par le fait qu'un mouvement rotatoire de l'organe de réglage (76), ayant lieu lors d'une injection (figure 28), n'est pas répercuté sur l'organe d'entraînement (128).

3. Appareil d'injection selon la revendication 2, dans lequel la partie filetée (122) est reliée, à l'organe d'entraînement (128), de telle sorte qu'un mouvement rotatoire dudit organe d'entraînement (128) soit répercuté sur ladite partie filetée (122), sans répercussion, toutefois, d'un coulissement axial dudit organe d'entraînement (128) par rapport au boîtier (50).

4. Appareil d'injection selon l'une des revendications 1 à 3, dans lequel un troisième filetage (62, 74) est interposé entre le boîtier (50) et l'organe de réglage (76), en vue du déplacement en longueur.

5. Appareil d'injection selon la revendication 4, dans lequel les pas des trois filetages sont coordonnés de façon telle que, au cours du réglage d'une dose d'injection, le mouvement de la pièce coulissante (66) dans une direction préétablie soit au moins de même ampleur que le mouvement de la partie filetée (122) dans cette direction.

6. Appareil d'injection selon la revendication 4 ou 5, dans lequel des tronçons de l'organe de réglage (76), situés dans la région du troisième filetage (62, 74), sont pourvus de valeurs graduées (78) en vue de l'affichage de la dose d'injection réglée.

7. Appareil d'injection selon la revendication 6, dans lequel le boîtier (50) est muni d'une fenêtre (52) pour afficher au moins l'une des valeurs graduées (78).

8. Appareil d'injection selon l'une des revendications 4 à 6, dans lequel le deuxième filetage (86, 94) et le troisième filetage (62, 74) présentent des directions de filetage identiques.

9. Appareil d'injection selon la revendication 8, dans lequel le pas du troisième filetage (62, 74) est d'une valeur supérieure à celle du pas du deuxième filetage (86, 94).

10. Appareil d'injection selon la revendication 8 ou 9, dans lequel la direction de filetage du premier filetage (100, 120) coïncide avec celle du troisième filetage (62, 74).

11. Appareil d'injection selon la revendication 10, dans lequel le pas du premier filetage (100, 120) est d'une valeur inférieure à celle du pas du troisième filetage (62, 74).

12. Appareil d'injection selon l'une des revendications 1 à 6, dans lequel la pièce coulissante (66) est pourvue, sur son côté proximal, d'une zone de contact (69) réalisée pour venir s'appliquer contre un côté distal (125) de la partie filetée (122) de telle sorte que, lors d'une injection, la tige (98) de piston soit animée d'un coulissement dans la direction proximale, par l'intermédiaire de ladite partie filetée (122), et que du liquide d'injection (110) soit exprimé du récipient (108).

13. Appareil d'injection selon la revendication 3 ou 4, dans lequel il est prévu un accouplement commandable (84, 136) autorisant, à l'état fermé, la répercussion d'un couple de rotation depuis l'organe de réglage (76) jusqu'à l'organe d'entraînement (128).

14. Appareil d'injection selon la revendication 15, dans lequel l'accouplement commandable (84, 136) est réalisé de façon telle que, lors du réglage d'une dose d'injection, il répercute le mouvement rotatoire de l'organe de réglage (76) sur l'organe d'entraînement (128).

**15.** Appareil d'injection selon la revendication 15 ou 16, dans lequel l'accouplement commandable (84, 136) est réalisé de façon telle que, lors d'une injection, il ne répercute pas un mouvement rotatoire de l'organe de réglage sur l'organe d'entraînement (128).

**16.** Appareil d'injection selon l'une des revendications 15 à 17, dans lequel l'accouplement commandable (84, 136) est réalisé de telle sorte qu'il soit ouvert suite à un processus s'opérant au début d'une injection.

**17.** Appareil d'injection selon l'une des revendications 15 à 18, dans lequel l'accouplement commandable (84, 136) peut être commandé, par l'opération d'injection, de façon telle qu'il retourne à l'état fermé après l'achèvement d'une injection, et demeure fermé au cours du réglage de dose, impliquant ainsi la répercussion sur la partie filetée (122), par l'intermédiaire dudit accouplement commandable (84, 136), d'une rotation de l'organe de réglage (76) effectuée en vue dudit réglage de dose.

**18.** Appareil d'injection selon l'une des revendications 1 à 6, dans lequel les pas des premier, deuxième et troisième filetages sont dimensionnés de façon telle que, au cours de l'opération de réglage, une rotation de l'organe de réglage (76) soit, pour l'essentiel, sans aucune influence sur la position de la tige (98) de piston vis-à-vis du boîtier (50).

**19.** Appareil d'injection selon l'une des revendications précédentes, dans lequel un ressort de torsion (148) est prévu et disposé de façon telle qu'il accumule de l'énergie lors d'un réglage de dose, et libère de l'énergie lors d'une opération d'injection.

**20.** Appareil d'injection selon la revendication 19, dans lequel le ressort de torsion (148) est interposé entre l'organe de réglage (76) et la pièce coulissante (66), une rotation relative, entre ces pièces, faisant varier le couple de rotation engendré par ledit ressort de torsion (148).

**21.** Appareil d'injection selon la revendication 20, dans lequel le ressort de torsion (148) est relié à la pièce coulissante (66) avec verrouillage rotatif, avec faculté de coulissement axial.

Fig.1

Fig.2

Fig.3

Fig.4

Fig.5

Fig.6

Fig.7

Fig.8

Fig.9

Fig.10

Fig.11

Fig.12

XIV

102

98

100

112

104

XIV

**Fig.13**

102

98

XIII

100

112

104

**Fig.14**

Fig.15

Fig.16

Fig.17

128

134 132

XX

XX

136

Fig.18

136

134 132

128

130

Fig.19

134 XIX

132

128

126

130 XIX

Fig.20

Fig.21

Fig.22

Fig.23

Fig.24

EP 1 610 848 B2

Fig.25

26

Fig.26

Fig.27

Fig.28

Fig.29

Fig.30

Fig.31

Fig.32

**EP 1 610 848 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0195959 A